# EUROPÄISCHE PATENTSCHRIFT

(11) **EP 1 609 735 B1**
(45) Veröffentlichungstag und Bekanntmachung des Hinweises auf die Patenterteilung: **20.09.2006**
(21) Anmeldenummer: 05012590.5
(22) Anmeldetag: 11.06.2005
(51) Int. Cl.: B65D 43/22, B05C 17/005, A61M 5/19

(54) **Kartusche für pastöse Materialien**
Cartridge for pasty materials
Cartouche destinée à des matières pâteuses

(30) Priorität: 23.06.2004 DE 102004030407
(43) Veröffentlichungstag der Anmeldung: 28.12.2005
(73) Patentinhaber: Heraeus Kulzer GmbH, 63450 Hanau (DE)
(72) Erfinder: Nehren, Klaus-Dieter, 41539 Dormagen (DE); Weber, Norbert, 50769 Köln (DE)
(74) Vertreter: Kühn, Hans-Christian

(56) Entgegenhaltungen:
- EP-A- 0 294 672
- EP-A- 0 521 776
- EP-A- 0 730 913
- DE-B- 1 225 928
- US-A- 4 392 589
- US-A- 5 439 131

## Beschreibung

Die Erfindung betrifft eine Kartusche für pastöse Materialien (US 4 392 589 A).

Derartige Kartuschen dienen nicht nur als Packmittel zum Lagern und Transportieren von Pasten, sondern sind auch als Bestandteil eines Systems aus Austraggerät, Spender- und Dosierkartusche und Mischer.

Dental-Abformmassen in Pastenform werden gewöhnlich in Schlauchbeuteln oder Kartuschen angeboten. Die in diesen Pasten enthaltenen Silikonöle, Vernetzer und Katalysatoren sind sehr kriech- und diffusionsfreudig. Insbesondere bei dentalen Abformmassen aus Vinylpolysiloxanen besteht daher das Problem der Abdichtung der Kartuschen.

Bei Doppelkartuschen mit für beide Komponenten gemeinsamen Mündungsteil - wie etwa in EP 0 232 733, EP 0 261 466, EP 0 294 672 beschrieben - kam es häufig zum Verstopfen einer der Komponentenauslasskanäle, weil Bestandteile der zweiten Komponente durch die direkte Verbindung dieser Kanäle überkriechen konnten.

Auch die Verwendung von Zapfen an den Verschlüssen - wie in EP 0 431 347 oder EP 0 578 897 beschrieben - konnte das Kriechen der Silikone nicht in allen Fällen vermeiden.

Durch den Einsatz von für die Einzelkomponenten getrennten runden Auslasskanälen (z.B. EP 0 730 913, EP 0 733 022, EP 1 125 641 und WO 00/21653) konnte das Überkriechen deutlich unterdrückt werden. Durch die dort verwendeten Bajonettverschlüsse zum Befestigen des Stopfens und des Mischers statischer oder dynamischer Art kommt es jedoch in der Praxis wieder dazu, dass das Silikonöl jetzt den gemeinsamen Verschluss und/oder den Bajonettring als Brücke nutzt, und wieder überkriechen kann.

Ein weiterer Nachteil dieser Konstruktionen ist, dass sie schwierig zu säubern sind. In der Regel werden bei diesen Systemen vom Anwender vor der ersten Anmischung beide Komponenten auf ein gleiches Förderniveau durch Austragen der einen etwas überfüllten Produktkammer gebracht. Dabei, und durch leichte Undichtigkeiten während eines Mischvorganges oder beim Mischerwechsel zwischen zwei Anwendungen wird häufig etwas Produkt verschmiert. Durch die dicht beieinander liegenden Auslasskanäle und die Formen der Bajonetthalterungen ist es nicht möglich, diese Verschmutzungen zu entfernen. Dadurch kann es dann bis zur nächsten Anwendung zur Verstopfung einer der Kanäle durch Kontamination kommen.

Es besteht ein Bedarf, eine verbesserte und vereinfachte Kartusche bereitzustellen.

Die Aufgabe wird durch die Merkmale des Anspruchs 1 gelöst durch eine Kartusche für pastöse Materialien mit zwei nebeneinander angeordneten zylindrischen Kammern, die je ein Fußende mit einer Öffnung und einen in Richtung der Zylinderachse in der jeweiligen Kammer verschiebbaren Kolben zur Ausbringung des in der Kammer enthaltenden Materials aufweisen, wobei die Kammern je ein dem Fußende gegenüber liegendes Kopfende aufweisen, welches je mindestens eine Auslassöffnung aufweist, wobei die Auslassöffnungen entweder je einen Verschlussstopfen aufweisen oder durch eine auf ihnen angeordnete gemeinsame Mischkammer miteinander verbunden sind, wobei die Mischkammer an ihrem den Auslassöffnungen zugewandten Ende einen Kragen aufweist, dadurch gekennzeichnet, dass am Kopfende einer der Kammern eine zwischen zwei Anschlagsstellungen schwenkbare Fixierplatte angeordnet ist, die in der einen Anschlagsstellung auf einer den Kammern abgewandten Oberfläche der beiden Verschlussstopfen und/oder des Kragens der Mischkammer angeordnet ist, um ein Lösen von Verschlussstopfen und/oder Mischkammer von den Auslassöffnungen zu verhindern, wobei die Fixierplatte in ihrer zweiten Anschlagsstellung nicht oberhalb der Oberfläche von Verschlussstopfen und/oder Kragen angeordnet ist. Die Fixierplatte überdeckt in ihrer ersten Anschlagstellung Verschlussstopfen und/oder Mischkammer zumindest teilweise in dem Maße, das notwendig ist, um ein Lösen dieser Teile von den Kammern zu verhindern, während sie Verschlussstopfen und/oder Mischkammer in ihrer zweiten Anschlagstellung zum Ablösen freigibt. Die Fixierplatte muß daher nicht notwendig unmittelbar auf der Oberfläche aufliegen, sofern Verschlussstopfen und/oder Mischkammer selbsttätig an den Auslässöffnungen festhalten. Bei Druck auf den Kolben wird jedoch der Druck auf die zum Ausgeben der Materialien auf die Auslassöffnungen gesteckte Mischkammer in der Regel so groß, dass sich diese an die Fixierplatte anlegt.

Vorzugsweise sind die Auslassöffnungen als Auslasskanäle ausgebildet. Zweckmäßig sind die Kopfenden der Kammern als Kopfplatten ausgebildet, wobei die Kopfplatten in einer gemeinsamen Ebene angeordnet sind. Es ist vorteilhaft, dass die den Kammern abgewandten Oberflächen der Verschlussstopfen und des Kragens der Mischkammer den gleichen Abstand zu den Kopfplatten aufweisen. Desweiteren ist es vorteilhaft, dass außerhalb des Schwenkbereichs der Fixierplatte auf den Kopfplatten Verstärkungsstege angeordnet sind. Insbesondere kann die Höhe der Verstärkungsstege über den Kopfplatten gleich dem Abstand der den Kammern abgewandten Oberfläche der Fixierplatte von den Kopfplatten sein, um eine ebene und stabile Auflage bei der Anwendung in einem Austraggerät zu erhalten. Die Verstärkungsstege sind axial umlaufend und/oder quer zum Umfang der Kammern verlaufend. Die Auslasskanäle beider Kammern werden mit separaten Stopfen verschlossen, die aber gemeinsam von der Schwenkplatte arretiert werden.

Die Form und Größe der Kartuschen ist durch die Anwendung vorgegeben. Es handelt sich insbesondere um Kartuschen zur Aufbewahrung und zum Ausbringen von dentalen Abformmaterialien.

Die Erfindung wird anhand einer Zeichnung näher erläutert. Darin zeigt:
- Fig. 1: die Kartusche mit geschlossenen Stopfen im Lager- und Transportzustand,
- Fig. 2: die Kartusche mit arretiertem Mischer (Mischkammer) für die Anwendung,
- Fig. 3: die Kartusche mit Fixierplatte in seitlicher Ansicht,
- Fig.s 4, 5: die Kartusche nach Fig. 3 in der Draufsicht mit geschlossener bzw. mit geöffneter Fixierplatte.

Die dargestellten Kartuschenkammern sind unterschiedlich groß und stellen Behälter für die weitverbreitete Anwendung im Dentalgebiet dar, bei der das Volumen einer Katalysatorpaste 1/5 desjenigen einer Basispaste beträgt, so dass bei Benutzung eine Mischung, zum Beispiel von Abdruckmassen, im vorbestimmten Verhältnis erfolgt.

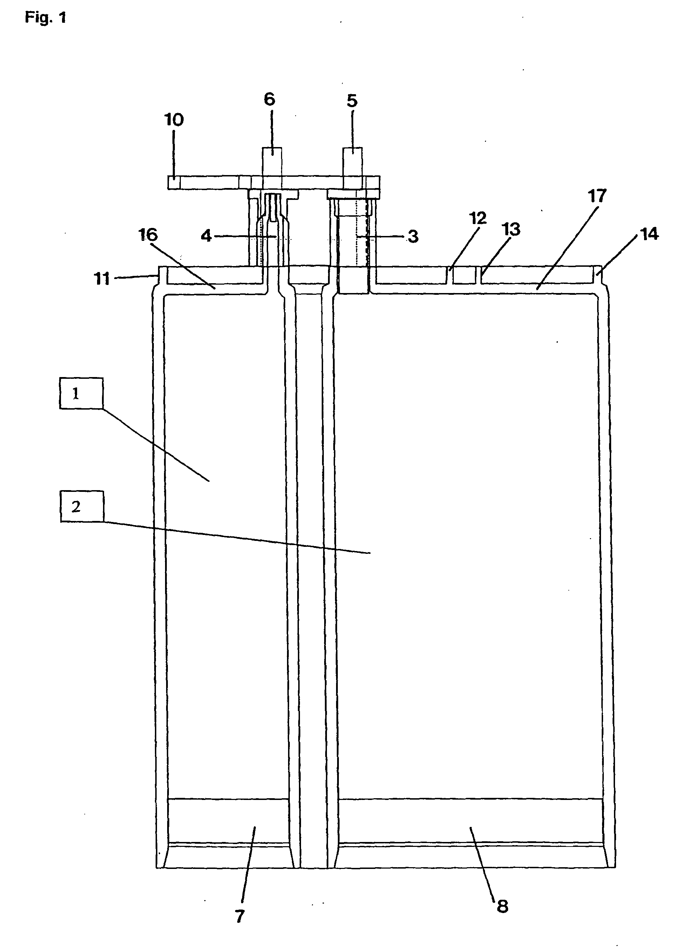

Fig. 1 zeigt die Kartusche mit zwei Kammern 1;2 mit durch Stopfen 5;6 geschlossenen Auslasskanälen 3;4 im Lager und Transportzustand. Die Stopfen sind mittels der Fixierplatte 10 auf den Auslasskanälen 3;4 gehalten. Der obere Abschluss der Kammern 1;2 wird durch Kopfplatten 16;17 gebildet, die in einer Ebene angeordnet sind. Die Kopfplatten 16;17 sind durch Verstärkungsstege 11;12;13;14 versteift. Im Unterteil der Kammern 1;2 sind verschiebbare Kolben 7;8 angeordnet, die als Mittel zum Ausbringen des pastösem Materials dienen.

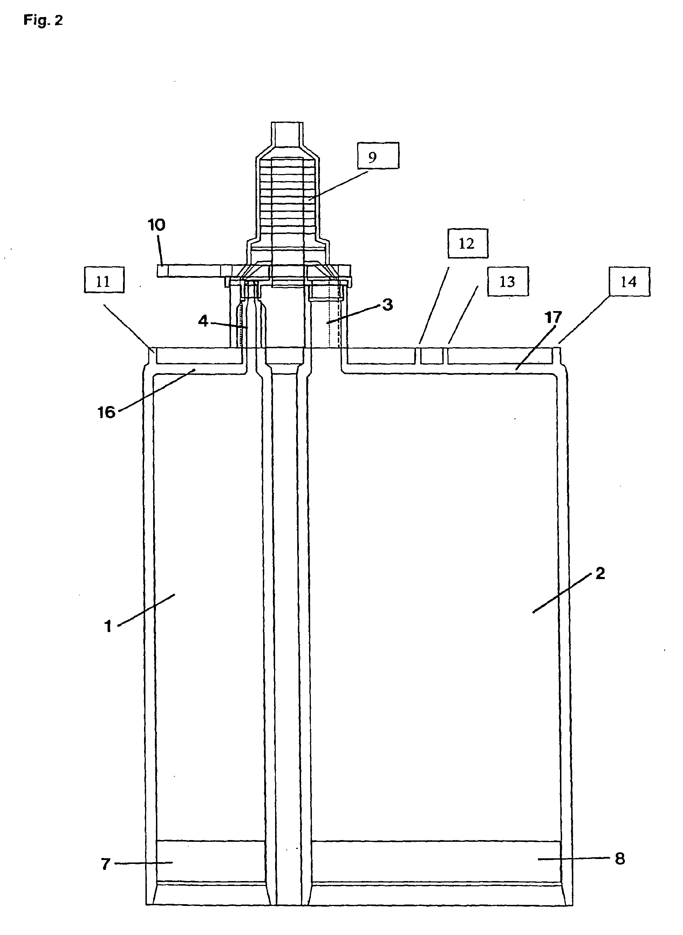

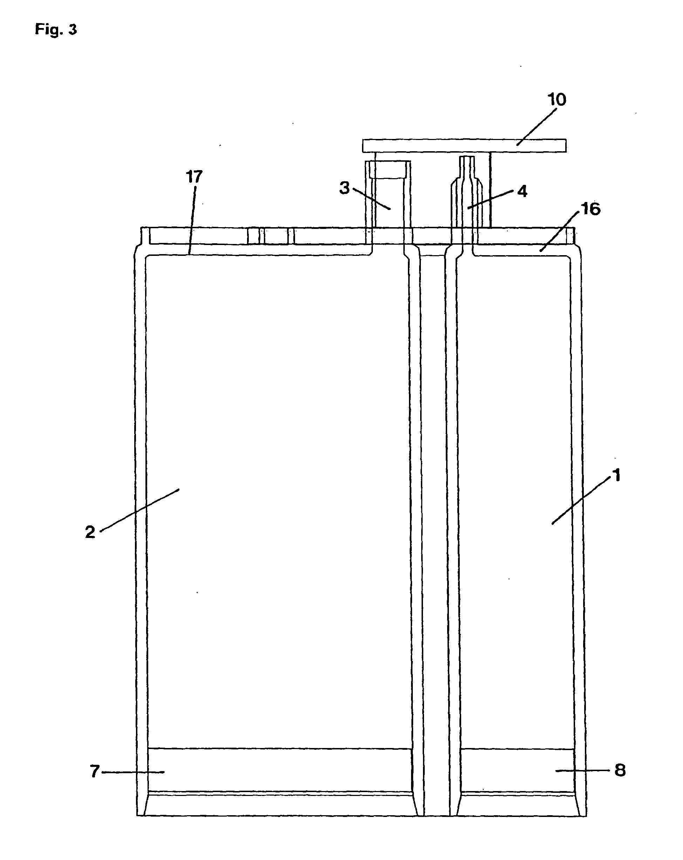

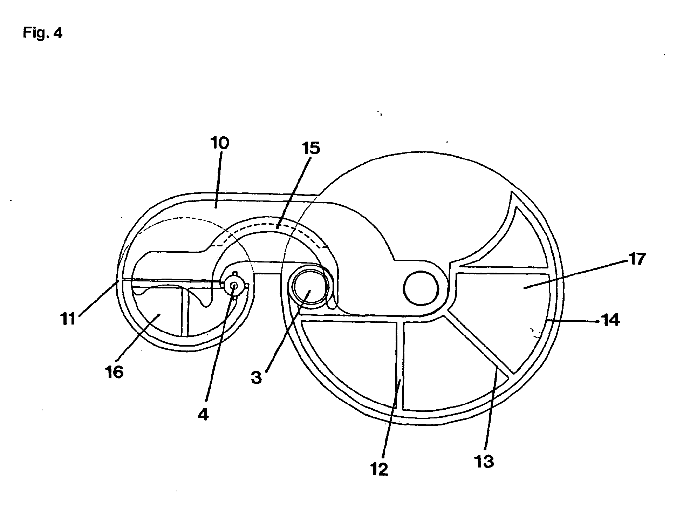

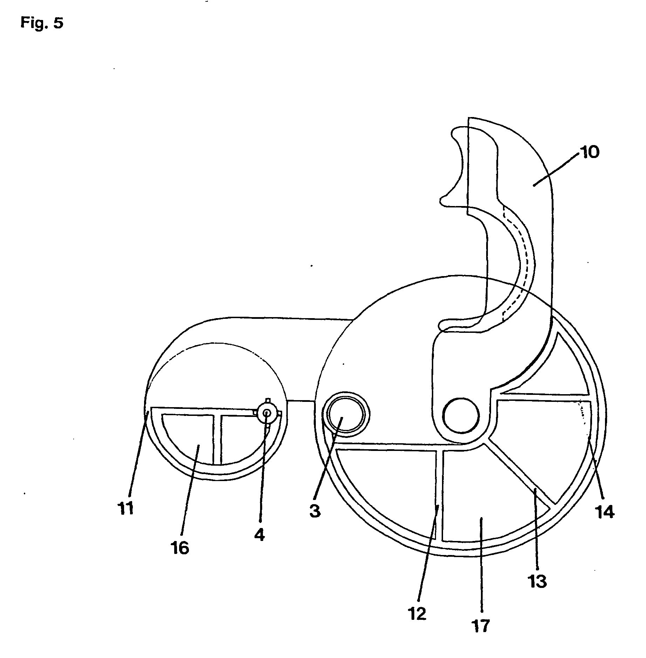

Fig. 2 zeigt die Kartusche mit arretierter Mischkammer 9, vorbereitet für die Anwendung. Die aufgesetzte Mischkammer 9 gibt die Auslasskanäle 3, 4 frei. Sie ist mittels der Fixierplatte 10 an der Kartusche gehalten. Fig. 3 zeigt die Kartusche mit Fixierplatte 10 in seitlicher Ansicht ohne Stopfen oder Mischkammer.

Fig.s 4, 5 zeigen die Kartusche in der Draufsicht mit geschlossener bzw. mit geöffneter Fixierplatte 10 in geöffnetem beziehungsweise geschlossenem Zustand. Stopfen oder Mischkammer sind in diesen Figuren der Einfachheit halber nicht dargestellt. Die beiden Anschlagstellungen der Fixierplatte 10 werden von Verstärkungsstegen gebildet. Oberkante der Verstärkungsstege und der Fixierplatte 10 bilden eine Ebene. Die Fixierplatte 10 weist an ihrer den Kammern 1;2 zugewandten Unterseite einen Vorsprung 15 auf zur Anlage an die seitliche Kante der Stopfen oder des Kragens der Mischkammer, so dass ein zuverlässiger Sitz der Fixierplatte bei korrekt aufgesetzten Stopfen/Mischkammer gewährleistet ist.

## Patentansprüche

1. Kartusche für pastöse Materialien mit zwei nebeneinander angeordneten zylindrischen Kammern (1;2), die je ein Fußende mit einer Öffnung und einen in Richtung der Zylinderachse in der jeweiligen Kammer (1;2) verschiebbaren Kolben (7;8) zur Ausbringung des in der Kammer (1;2) enthaltenden Materials aufweisen, wobei die Kammern (1;2) je ein dem Fußende gegenüber liegendes Kopfende aufweisen, welches je mindestens eine Austassöffnung aufweist, wobei die Auslassöffnungen entweder je einen Verschlussstopfen (5;6) aufweisen oder durch eine auf ihnen angeordnete gemeinsame Mischkammer (9) miteinander verbunden sind, wobei die Mischkammer (9) an ihrem den Auslassöffnungen zugewandten Ende einen Kragen aufweist, **dadurch gekennzeichnet, dass** am Kopfende einer der Kammern (1;2) eine zwischen zwei Anschlagsstellungen schwenkbare Fixierplatte (10) angeordnet ist, die in der einen Anschlagsstellung auf einer den Kammern (1;2) abgewandten Oberfläche der beiden Verschlussstopfen (5;6) und/oder des Kragens der Mischkammer (9) angeordnet ist, um ein Lösen von Verschlussstopfen (5;6) und/oder Mischkammer (9) von den Austassöffnungen zu verhindern, wobei die Fixierplatte (10) in ihrer zweiten Anschlagsstellung nicht oberhalb der Oberfläche von Verschlussstopfen (5;6) und/oder Kragen angeordnet ist.

2. Kartusche nach Anspruch 1, **dadurch gekennzeichnet, dass** die Auslassöffnungen als Auslasskanäle (3;4) ausgebildet sind.

3. Kartusche nach Anspruch 1 oder 2, **dadurch gekennzeichnet, dass** die Kopfenden der Kammern (1 ;2) als Kopfplatten ausgebildet sind, wobei die Kopfplatten in einer gemeinsamen Ebene angeordnet sind.

4. Kartusche nach Anspruch 3, **dadurch gekennzeichnet, dass** die den Kammern (1;2) abgewandten Oberflächen der Verschlussstopfen (5;6) und des Kragens der Mischkammer (9) den gleichen Abstand zu den Kopfplatten aufweisen.

5. Kartusche nach Anspruch 3 oder 4, **dadurch gekennzeichnet, dass** außerhalb des Schwenkbereichs der Fixierplatte (10) auf den Kopfplatten Verstärkungsstege (11;12;13;14) angeordnet sind.

6. Kartusche nach Anspruch 5, **dadurch gekennzeichnet, dass** die Höhe der Verstärkungsstege (11;12;13;14) über den Kopfplatten gleich dem Abstand der den Kammern (1 ;2) abgewandten Oberfläche der Fixierplatte (10) von den Kopfplatten ist.

## Claims

1. Cartridge for highly viscous materials with two adjacently arranged cylindrical chambers (1;2) each comprising one base end with an opening and one piston (7;8) displaceable in the direction of the cylinder axis in the corresponding chamber (1 ;2) for bringing out the material contained in the chamber (1;2), with the chambers (1;2) having a head end lying opposite of the base end, the head end having each at least one outlet opening, with the outlet openings each either comprising one closing plug (5;6) each, or being connected with each other through a joint mixing chamber (9) arranged on them, with the mixing chamber (9), at its end facing the outlet openings, comprising a collar, **characterized in that** - at the head end of one of the chambers (1;2) - a fixing plate (10) swivable between two stop positions being arranged which is provided in the one stop position on a surface - facing away from the chambers (1;2) - of the two closing plugs (5;6) and/or the collar of the mixing chamber (9) to prevent a loosening of the closing plugs (5;6) and/or of the mixing chamber (9) from the outlet openings, with the fixing plate (10) in its second stop position not being arranged above the surface of the closing plugs (5;6) and/or the collar.

2. Cartridge according to claim 1, **characterized in that** the outlet openings are formed as outlet channels (3;4).

3. Cartridge according to claim 1 or 2, **characterized in that** the head ends of the chambers (1 ;2) are formed as head plates, with the head plates being arranged in a joint level.

4. Cartridge according to claim 3, **characterized in that** the surfaces of closing plugs (5;6) and collar of the mixing chamber (9) which are facing away from the chambers (1;2) have the same distance to the head plates.

5. Cartridge according to claim 3 or 4, **characterized in that** - outside of the swivel range of the fixing plate (10) - reinforcement stays (11;12;13;14) are provided on the head plates.

6. Cartridge according to claim 5, **characterized in that** the height of the reinforcement stays (11;12;13;14) above the head plates is identical to the distance between the fixing plate (10) surface - facing away from the chambers (1;2) - and the head plates.

## Revendications

1. Cartouche pour matériaux pâteux avec deux chambres cylindriques (1 ; 2) disposées l'une à côté de l'autre qui présentent chacune une extrémité de pied avec une ouverture et un piston (7 ; 8) pouvant être déplacé dans la direction de l'axe cylindrique dans la chambre respective (1 ; 2) en vue d'extraire le matériau contenu dans la chambre (1 ; 2), les chambres (1 ; 2) présentant chacune une extrémité de tête opposée à l'extrémité de pied qui présente chacune au moins une ouverture de sortie, les ouvertures de sortie présentant soit chacune un bouchon de fermeture (5 ; 6), soit étant reliées ensemble par une chambre de mélange commune (9) disposée sur elles, la chambre de mélange (9) présentant sur son extrémité tournée vers les ouvertures de sortie un col, **caractérisée en ce qu'**une plaque de fixation (10) pivotante entre deux positions de butée qui est disposée dans une position de butée sur une surface détournée des chambres (1 ; 2) des deux bouchons de fermeture (5 ; 6) et/ou du col de la chambre de mélange (9), est disposée sur l'extrémité de tête d'une des chambres (1 ; 2) afin d'empêcher un desserrage des bouchons de fermeture (5 ; 6) et/ou de la chambre de mélange (9) des ouvertures de sortie, la plaque de fixation (10) n'étant pas disposée dans sa deuxième position de butée au-dessus de la surface des bouchons de fermeture (5 ; 6) et/ou du col.

2. Cartouche selon la revendication 1, **caractérisée en ce que** les ouvertures de sortie sont réalisées en tant que canaux de sortie (3 ; 4).

3. Cartouche selon la revendication 1 ou 2, **caractérisée en ce que** les extrémités de tête des chambres (1 ; 2) sont réalisées en tant que plaques de tête, les plaques de tête étant disposées dans un plan commun.

4. Cartouche selon la revendication 3, **caractérisée en ce que** les surfaces détournées des chambres (1 ; 2) des bouchons de fermeture (5 ; 6) et du col de la chambre de mélange (9) présentent la même distance aux plaques de tête.

5. Cartouche selon la revendication 3 ou 4, **caractérisée en ce que** des nervures de renforcement (11 ; 12 ; 13 ; 14) sont disposées en dehors de la zone de pivotement de la plaque de fixation (10) sur les plaques de tête.

6. Cartouche selon la revendication 5, **caractérisée en ce que** la hauteur des nervures de renforcement (11 ; 12 ; 13 ; 14) au-dessus des plaques de tête est égale à la distance de la surface détournée des chambres (1 ; 2) de la plaque de fixation (10) des plaques de tête.
